# FASCICULE DE BREVET EUROPEEN

(11) **EP 3 867 257 B1**
(45) Date de publication et mention de la délivrance du brevet: **18.10.2023**
(21) Numéro de dépôt: 19789984.2
(22) Date de dépôt: 21.10.2019
(51) Int. Cl.: C07F 9/54, C07C 17/16, C07F 9/6596

(54) **ADDUITS DE TRIPHENYLPHOSPHINE ET DE TRIPHENYLPHOSPHITE ET LEUR UTILISATION POUR LA BROMATION D'ALCOOLS PRIMAIRES**
ADDUKTE VON TRIPHENYLPHOSPHIN UND TRIPHENYLPHOSPHIT UND DEREN VERWENDUNG ZUR BROMIERUNG PRIMÄRER ALKOHOLE
ADDUCTS OF TRIPHENYLPHOSPHINE AND TRIPHENYLPHOSPHITE AND THEIR USE FOR THE BROMINATION OF PRIMARY ALCOHOLS

(30) Priorité: 19.10.2018 FR 1859688
(43) Date de publication de la demande: 25.08.2021
(73) Titulaire: Melchior Material and Life Science France, 64170 Lacq (FR)
(72) Inventeur: GUERRET, Olivier, 46170 Pern (FR)
(74) Mandataire: Regimbeau
(86) Numéro de dépôt international: PCT/EP2019/078597
(87) Numéro de publication internationale: WO 2020/079281

(56) Documents cités:
- FR-A1- 3 016 166
- US-A- 6 063 971

## Description

La présente invention concerne l'utilisation de nouveaux adduits de formule générale (I) TPPₓTPOP₍₁₋ₓ₎, où TPP désigne la triphényl phosphine, TPOP le triphényl phosphite et x un nombre réel compris entre 0,05 et 0,9. Ces nouveaux adduits sont utiles pour la bromation d'alcools primaires, en particulier pour la bromation d'alcools primaires contenant un cycle tendu à 3 ou 4 atomes de carbone, tels que le cyclopropyl-méthanol ou le cyclobutyl-méthanol.

Plus particulièrement ces adduits sont utilisés avantageusement dans un procédé d'obtention de composés bromés de haute pureté dans des conditions de synthèse permettant une grande productivité des installations industrielles, en particulier du bromo-méthyl-cyclopropane **(2a)** et du bromo-méthylcyclobutane **(2b)** de haute pureté, à partir respectivement du cyclopropyl-méthanol **1a** et du cyclobutyl-méthanol **1b.** Ces composés bromés, en particulier les composés **2a** et **2b,** sont des intermédiaires de synthèse essentiels à de nombreuses substances actives.

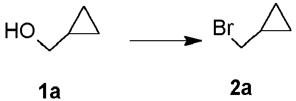

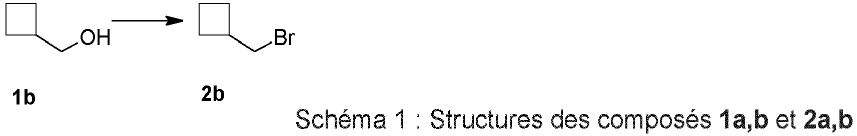

### CONTEXTE DE L'INVENTION :

Du fait de la grande réactivité des cycles tendus cyclopropyle et cyclobutyle, il est souvent préférable pour l'homme de l'art d'utiliser ces intermédiaires dans les dernières étapes de préparation des substances actives. Ce fait a pour conséquence que la pureté des molécules de bromo-méthyl-cyclopropane (BMCP) et de bromo-méthylcyclobutane (BMCB) est essentielle puisque qu'elle influence directement le profil d'impureté des substances actives. Les impuretés les plus difficiles à contrôler sont pour le BMCP : le 4-brombut-1-ène et le bromocyclobutane et pour le BMCB : le 5-bromopent-1-ène et le bromocyclopentane, car elles ont des points d'ébullition très proches des molécules cibles. Il est donc essentiel que le contrôle de ces impuretés ait lieu avant la distillation grâce aux conditions de synthèse.

Récemment deux brevets ont révélé une méthode de production du BMCP. Dans le brevet FR3010997, un procédé impliquant dans cet ordre le triphényl phosphite dilué dans le diméthyl formamide, le dibrome puis de carbinol est décrit. Les inventeurs évoquent le passage par un complexe permettant l'obtention d'un BMCP présentant un faible niveau d'impuretés et une bonne productivité.

Le triphényl phosphite, encore noté TPOP, répond à la formule (II) suivante :

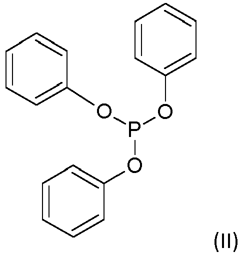

Dans le brevet FR3016166, la demanderesse décrit un procédé impliquant les mêmes réactifs que précédemment utilisés dans des conditions différentes (température, dilution) mais conduisant aussi à un BMCP de très faible niveau d'impuretés. L'intermédiaire réactionnel dans le cas de ce procédé est un sel de phosphonium connu pour conduire sélectivement aux bromations d'alcool primaires ou secondaires (voir dans O. Castro, Organic Reactions, vol. 29 William G Sauben & al., 1983, Organic Reactions, Inc publié par John Wiley & Sons Inc. pages 24-25). Un procédé de fabrication du bromo-méthyl-cyclopropane est décrit dans le brevet US 6063971.

Parmi les travaux antérieurs à ces demandes de brevet, le brevet US 61913001 décrivait une méthode assez voisine à la différence que le triphényl phosphite est remplacé par la triphényl phosphine et que tous les réactifs sont introduits en même temps dans le réacteur. Ce procédé, moyennant un excès de dibrome significatif, conduit au BMCP ultra pur.

La triphényl phosphine, encore notée TPP, répond à la formule (III) suivante

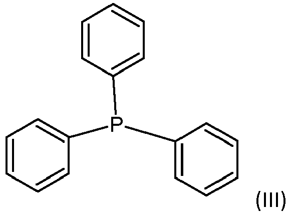

Or, du fait de l'évolution des besoins de l'industrie pharmaceutique, les spécifications sont devenues de plus en plus dures. Ainsi de 0,5% en poids, par rapport au poids total, de 4-bromobut-1-ène toléré dans le BMCP, les fabricants de principes actifs ont fixé de nouvelles limites à 0,2% en poids, par rapport au poids total.

Le brevet US 61913001 décrit une méthode permettant d'atteindre ces niveaux de spécification ce qui traduit un caractère sélectif plus grand de l'adduit formé entre la triphényl phosphine et le dibrome que celui de l'adduit formé entre le triphényl phosphite et le dibrome. Cependant le procédé de ce brevet US 61913001 n'est pas productif du fait de la faible solubilité de la triphényl phosphine dans le DMF. Un tel problème est illustré dans le troisième exemple du brevet FR3016166.

Par ailleurs, dans un secteur de plus en plus soumis à la compétition internationale, augmenter la productivité réactionnelle est un atout essentiel pour garder des parts de marché.

De manière surprenante, la demanderesse a trouvé que l'utilisation d'adduit de formule générale TPPₓTPOP(₁₋ₓ) où x est un nombre réel compris entre 0,05 et 0,9 dans un procédé de bromation avec du dibrome permet d'avoir des rendements supérieurs et des productivités améliorées par rapport à l'utilisation soit de TPP seule soit de TPOP seul.

La réactivité des cycles tendus étant particulièrement délicate, on comprendra aisément que l'utilisation de ces adduits dans un procédé de bromation peut s'appliquer à la bromation de l'ensemble des alcools primaires.

### BREVE DESCRIPTION DE L'INVENTION

Selon un premier mode de réalisation, l'invention concerne un adduit de formule générale (I)

TPPₓTPOP₍₁₋ₓ₎ (I)

où
- TPP désigne la triphényl phosphine ;
- TPOP désigne le triphényl phosphite ; et
- x est un nombre réel compris entre 0,05 et 0,9.

L'invention vise encore un adduit selon le premier mode de réalisation, pour lequel x est compris entre 0,05 et 0,5, voire 0,1 et 0,5.

L'invention vise aussi selon un troisième mode de réalisation, un procédé de préparation de l'adduit selon le mode de réalisation 1 ou 2, dans lequel on mélange x part en moles de triphényl phosphine et (1-x) part en moles de triphényl phosphite à une température Tx comprise entre 20°C et 50°C.

L'invention vise aussi, selon un quatrième mode de réalisation de l'invention, un procédé de préparation selon le mode de réalisation 3, dans lequel Tx vaut 25°C et x<0,5.

Dans un cinquième mode de réalisation, l'invention vise l'utilisation de l'adduit selon le mode de réalisation 1 ou 2 ou de l'adduit obtenu par le procédé selon le mode de réalisation 3 ou 4 en tant qu'agent liquide de bromation d'alcool primaire.

L'invention vise aussi dans un sixième mode de réalisation, une utilisation selon le mode de réalisation précédent, dans laquelle l'alcool primaire comprend un cycle hydrocarboné à 3 ou 4 atomes de carbone, en particulier l'alcool primaire est le cyclopropyl-méthanol ou le cyclobutyl-méthanol.

Selon un septième mode de réalisation, l'invention concerne un procédé de préparation d'un composé bromé (A) par bromation d'alcool primaire comprenant les étapes suivantes :
1. mélanger (a) 1 équivalent molaire de l'adduit (I) selon l'invention ou de l'adduit obtenu par le procédé selon l'invention et (b) 1 à 3 volumes de solvant polaire ; puis
2. ajouter le dibrome :
   2.1 refroidir sous agitation la solution obtenue suite à l'étape 1) à une température comprise entre 0°C et 12°C, puis
   2.2à la solution refroidie obtenue suite à l'étape 2.1), ajouter entre 1,05 et 1,25 équivalent molaire de dibrome en maintenant la température du milieu réactionnel en dessous de 12°C ; puis
3. ajouter l'alcool primaire :
   3.1 suite à l'étape 2.2), abaisser la température du milieu réactionnel à une valeur comprise entre 0°C et -15°C ;
   3.2au milieu réactionnel refroidi obtenu suite à l'étape 3.1), ajouter entre 0,95 et 1 équivalent molaire d'alcool primaire, puis
4. récupérer le composé bromé (A).

C'est aussi un objet de l'invention, dans un huitième mode de réalisation, que de fournir un procédé selon le mode de réalisation 7, dans lequel le solvant polaire est choisi parmi le diméthyl formamide, le diméthyl sulfoxyde, le sulfolane, le dichlorométhane, le tétrahydrofurane, le diméthylglycol éther, le dichloroéthane, l'acétonitrile, et leurs mélanges.

Selon un neuvième mode de réalisation, l'invention concerne aussi un procédé selon le mode de réalisation 7 ou 8, dans lequel lors de l'étape 2.2) le milieu réactionnel est maintenu à une température comprise entre 0°C et 12°C.

Dans un dixième mode de réalisation, l'invention vise encore un procédé selon l'un quelconque des modes de réalisation 7 à 9, dans lequel, lors de l'étape 3.2), le milieu réactionnel est maintenu à une température comprise entre 0°C et - 15°C, avantageusement comprise entre 0°C et -5°C.

Selon un onzième mode de réalisation, l'invention concerne un procédé selon l'un quelconque des modes de réalisation 7 à 10, dans lequel l'alcool primaire comprend un cycle hydrocarboné à 3 ou 4 atomes de carbone.

Selon un douzième mode de réalisation, l'invention vise aussi un procédé selon l'un quelconque des modes de réalisation 7 à 11, dans lequel l'alcool primaire est le cyclopropyl carbinol ou e cyclobutyl carbinol.

Selon un treizième mode de réalisation, l'invention vise un procédé selon le mode de réalisation 12, dans lequel l'alcool primaire est le cyclopropyl carbinol et le composé bromé récupéré comprend, en poids par rapport au poids total, moins de 0,2% d'impuretés choisies parmi le 4-bromobut-1-ène et le bromocyclobutane.

Selon un quatorzième mode de réalisation l'invention fournit aussi un adduit de formule générale (II) :

TPPₓTPOP₍₁₋ₓ₎Br₂₍ₚ₎ (II)

où
- TPP désigne la triphényl phosphine ;
- TPOP désigne le triphényl phosphite ; et
- x est un nombre réel compris entre 0,05 et 0,9,
- p est égal à 1 ou 2.

C'est enfin un quinzième mode de réalisation de l'invention que de viser l'utilisation de l'adduit selon le mode de réalisation 14 pour la bromation d'alcool primaire, en particulier le cyclopropylméthanol ou le cyclobutylméthanol.

Dans la présente description, sauf indication expresse différente, tous les pourcentages (%) indiqués sont des % en masse.

Dans la présente description, sauf indication expresse différente, la pression est la pression atmosphérique.

Dans la présente description, par « température ambiante », on entend une température comprise entre 15°C et 40°C, de préférence entre 20°C et 30°C, notamment d'environ 25°C.

D'autre part, tout intervalle de valeurs désigné par l'expression "entre a et b" représente le domaine de valeurs allant de a jusqu'à b (c'est-à-dire incluant les bornes strictes a et b).

Dans la présente description, l'« adduit » de formule (I) désigne le produit d'addition résultant de l'addition de x part en moles de triphényl phosphine et de (1-x) part en moles de triphényl phosphite. En particulier, il désigne le produit d'addition résultant de l'addition de x part en moles de triphényl phosphine dans (1-x) part en moles de triphényl phosphite à une température Tx comprise entre 20°C et 50°C. Plus particulièrement, l'adduit selon la présente invention est un mélange consistant en x part en moles de triphényl phosphine et (1-x) part en moles de triphényl phosphite. Ce mélange représentant l'adduit est utilisable et utilisé comme réactif, ou agent, liquide dans un procédé de bromation d'alcool primaire, en particulier le cyclopropylméthanol ou le cyclobutylméthanol.

Un autre objet de l'invention est les adduits obtenus en faisant réagir du dibrome et les adduits de formule (I), TPOP₍₁₋ₓ₎TPPₓ, utilisables en tant qu'agents de bromation en particulier des alcools primaires.

Les quantités exprimées dans la présente description en équivalents correspondent à des équivalents molaires par rapport à l'alcool primaire utilisé, en particulier le cyclopropylméthanol ou le cyclobutylméthanol.

### DESCRIPTION DETAILLEE DE L'INVENTION

L'invention a pour objet un adduit de formule générale (I)

TPPₓTPOP₍₁₋ₓ₎ (I)

où
- TPP désigne la triphényl phosphine ;
- TPOP désigne le triphényl phosphite ; et
- x est un nombre réel compris entre 0,05 et 0,9.

La TPP répond à la formule (III) décrite précédemment et le TPOP répond à la formule (II) décrite précédemment.

Avantageusement, x est compris entre 0,05 et 0,5, voire entre 0,2 et 0,5.

Avantageusement, x est strictement inférieur à 0,5.

L'adduit de formule générale (I) est avantageusement liquide à une température comprise entre -20°C et 50°C, le rendant ainsi adapté à une utilisation en synthèse industrielle.

L'adduit de formule générale TPPₓTPOP₍₁₋ₓ₎ est obtenu en mélangeant x part en moles de triphényl phosphine et (1-x) part en moles de triphényl phosphite. En particulier, l'adduit de formule générale TPPₓTPOP₍₁₋ₓ₎ est obtenu en mélangeant x part en moles de triphényl phosphine dans (1-x) part en moles de triphényl phosphite. Pour des raisons de viscosité, ce mélange a avantageusement lieu à une température Tx comprise entre 20°C et 50°C, plus avantageusement comprise entre 25°C et 50°C.

De préférence, on utilisera entre 0,2 et 0,5 part en moles de triphényl phosphine et entre 0,8 et 0,5 part en moles de triphényl phosphite.

Autrement formulée, l'invention a pour objet un adduit de formule générale (I)

TPPₓTPOP₍₁₋ₓ₎ (I)

où
- TPP désigne la triphényl phosphine ;
- TPOP désigne le triphényl phosphite ; et
- x est un nombre réel compris entre 0,05 et 0,9,
obtenu en mélangeant x part en moles de triphényl phosphine et (1-x) part en moles de triphényl phosphite.

De manière particulière, et compte tenu du fait que la triphényl phosphine est solide et que le triphénylphosphite est liquide à température ambiante (env 20 à 25°C) et pression atmosphérique (env 760 mm de Hg (env 101325 Pa)), l'adduit de formule générale (I) selon l'invention est obtenu en solubilisant x part de triphényl phosphine dans (1-x) part en moles de triphényl phosphite.

La triphénylphosphine étant solide (et peu soluble dans le milieu réactionnel), une perte de productivité est possible. Cette différence de solubilité-miscibilité est d'autant plus importante du fait des températures basses employées afin d'obtenir une bonne sélectivité. Le triphénylphosphite joue donc ainsi le rôle de solvant (liquide à température ambiante) et de réactif dans la bromation. C'est ainsi que l'on peut penser que l'adduit (I) selon l'invention représente un adduit de solvatation, car en effet la triphénylphosphine est solvatée par le triphénylphosphite lors de cette solubilisation.

L'ajout de la triphénylphosphine dans le triphénylphosphite permet donc la formation d'un adduit de formule générale triphénylphosphine_{(X)}-triphénylphosphite_{(1-X)} aussi noté TPP_{(X)}TPOP_{(1-X)}.

La masse molaire Mₓ de l'adduit est calculée selon la formule : Mₓ= x*262 + (1-x)*310 g/mol.

La demanderesse ne formulera pas d'hypothèse quant à la structure de cet adduit (I), mais suppose qu'un tel adduit, une fois mis en contact avec le dibrome va donner une structure du type : 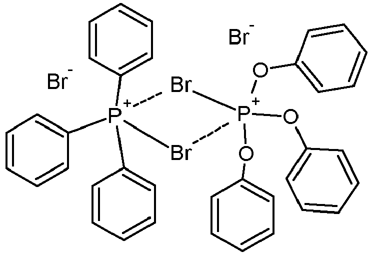 qui peut avoir des réactivités différentes à celles obtenues avec le dibrome et la TPP (x=1) ou avec le dibrome et le TPOP (x=0). La découverte des différences de réactivité montre bien qu'il n'est pas évident pour l'homme du métier de prévoir le comportement de l'adduit selon l'invention par rapport aux produits purs, TPP ou TPOP.

C'est aussi un autre objet de la présente invention que de fournir un adduit de formule générale (II) :

TPPₓTPOP₍₁₋ₓ₎Br₂₍ₚ₎ (II)

où
- TPP désigne la triphényl phosphine ;
- TPOP désigne le triphényl phosphite ; et
- x est un nombre réel compris entre 0,05 et 0,9,
- p est égal à 1 ou 2.

Un tel adduit selon la formule (II) peut être obtenu en mélangeant 1 équivalent molaire de l'adduit de formule (I), optionnellement dilué dans 1 à 3 volumes d'un solvant polaire, avec p équivalent molaire de Br₂.

Le solvant polaire est avantageusement choisi parmi le diméthyl formamide, le diméthyl sulfoxyde, le sulfolane, le dichlorométhane, le tétrahydrofurane, le diméthylglycol éther, le dichloroéthane, l'acétonitrile, et leurs mélanges.

La présente invention concerne encore l'utilisation d'un adduit de formule générale (II) pour la bromation d'alcool primaire, en particulier le cyclopropylméthanol ou le cyclobutylméthanol.

De manière surprenante, il a été découvert que cet adduit (I) est un excellent réducteur, particulièrement adapté à la bromation d'alcool primaire, en particulier d'alcool primaire comprenant un cycle hydrocarboné à 3 ou 4 atomes de carbone, tel que le cyclopropyl carbinol ou le cyclobutyl carbinol. Cet adduit (I) permet d'avoir des rendements supérieurs et des productivités améliorées par rapport à l'utilisation de TPP seule ou de TPOP seul.

L'invention a également pour objet un procédé de préparation de l'adduit (I), ou de l'adduit (II), selon l'invention, dans lequel on mélange x part en moles de triphényl phosphine et (1-x) part en moles de triphényl phosphite, à une température Tx comprise entre 20°C et 50°C. En particulier, on ajoute x part en moles de triphényl phosphine dans (1-x) part en moles de triphényl phosphite, à une température Tx comprise entre 20°C et 50°C.

La température Tx de mélange pour la formation de cet adduit est choisie de manière à ce que le mélange se fasse sans problème de viscosité. Ainsi, pour au plus 0,5 part en moles de triphényl phosphine, conduisant à un adduit de formule (I) dans lequel x est strictement inférieur à 0,5, une température de 25°C ou inférieure à 25°C peut être utilisée.

Un adduit selon la formule (II) pourra être obtenu en mélangeant 1 équivalent molaire de l'adduit de formule (I) ainsi obtenu, optionnellement dilué dans 1 à 3 volumes d'un solvant polaire, avec p équivalent molaire de Br₂. L'invention vise aussi l'utilisation d'un tel adduit (II) ou d'un adduit (II) ainsi obtenu, en tant qu'agent liquide, ou réactif liquide, pour la bromation d'alcool primaire.

Le solvant polaire est avantageusement choisi parmi le diméthyl formamide, le diméthyl sulfoxyde, le sulfolane, le dichlorométhane, le tétrahydrofurane, le diméthylglycol éther, le dichloroéthane, l'acétonitrile, et leurs mélanges.

De manière avantageuse, dans la formule (I) ou (II), x sera compris entre 0,05 et 0,8, plus particulièrement entre 0,05 et 0,5, voire encore 0,1 à 0,5, voire encore entre 0,2 et 0,5. Il a été remarqué que, compte tenu de la solubilité de la triphénylphosphine à température ambiante, x sera avantageusement compris entre 0,05 et 0,4, voire 0,05 et 0,4, voire encore 0,1 et 0,4. En effet, au-delà de 0,4 et à température ambiante, on pourra rencontrer difficulté de solubilité. Aussi de manière particulièrement préférée, x est compris entre 0,05 et 0,3, voire entre 0,1 et 0,3, ou encore entre 0,2 et 0,3.

Une telle gamme de valeurs de x permet de minimiser l'alcool primaire résiduel, de maximiser la quantité de produit fini bromé, et de limiter à des valeurs très basses (voire non détectables) les impuretés telles que le 4-bromo-1-butène et le bromocyclobutane.

L'invention a également pour objet l'utilisation de l'adduit (I) ou (II) selon l'invention ou de l'adduit obtenu par le procédé selon l'invention en tant qu'agent liquide, ou réactif liquide, pour la bromation d'alcool primaire.

L'alcool primaire comprend avantageusement un cycle hydrocarboné à 3 ou 4 atomes de carbone, en particulier l'alcool primaire est le cyclopropyl-méthanol ou le cyclobutyl-méthanol.

Ainsi, l'invention a également pour objet un procédé de préparation d'un composé bromé (A) par bromation d'alcool primaire comprenant les étapes suivantes :
1 mélanger (a) 1 équivalent molaire de l'adduit (I) selon l'invention ou de l'adduit obtenu par le procédé selon l'invention et (b) 1 à 3 volumes de solvant polaire ; puis
2 ajouter le dibrome :
   2.1 refroidir sous agitation la solution obtenue suite à l'étape 1) à une température comprise entre 0°C et 12°C, puis
   2.2à la solution refroidie obtenue suite à l'étape 2.1), ajouter entre 1,05 et 1,25 équivalent molaire de dibrome en maintenant la température du milieu réactionnel en dessous de 12°C ; puis
3 ajouter l'alcool primaire :
   3.1 suite à l'étape 2.2), abaisser la température du milieu réactionnel à une valeur comprise entre 0°C et -15°C ;
   3.2au milieu réactionnel refroidi obtenu suite à l'étape 3.1), ajouter entre 0,95 et 1 équivalent molaire d'alcool primaire, puis
4 récupérer du composé bromé (A).

Dans une première étape, un équivalent d'adduit (I) est mélangé dans un solvant polaire. Le volume de dilution est compris entre 1 et 3 volumes par rapport à l'adduit (I). L'ajout de solvant permet de diluer le milieu réactionnel en vue de l'ajout subséquent de l'alcool. Dans le cas de la seule obtention de l'adduit (II), ledit solvant est optionnel comme expliqué ci-avant.

Le solvant polaire est avantageusement choisi parmi le diméthyl formamide, le diméthyl sulfoxyde, le sulfolane, le dichlorométhane, le tétrahydrofurane, le diméthylglycol éther, le dichloroéthane, l'acétonitrile, et leurs mélanges.

L'étape 1) se fait avantageusement à température ambiante.

Dans une deuxième étape de ce procédé, le dibrome est ajouté à la solution comprenant l'adduit (I), avantageusement dilué dans un solvant polaire, permettant la formation de l'adduit (II).

Cette deuxième étape comprend tout d'abord une étape 2.1) de refroidissement, avant tout ajout de dibrome, puis le dibrome est ajouté (étape 2.2)).

Lors de cette étape 2.2), la température du mélange réactionnel est avantageusement maintenue entre 0°C et 12°C. Ainsi, le dibrome est avantageusement ajouté lentement, c'est-à-dire à une vitesse telle que la température du milieu réactionnel ne dépasse pas 12°C.

Avantageusement, suite à l'étape 2.2), c'est-à-dire après complétion de l'ajout de dibrome, et avant l'étape 3), le milieu réactionnel est agité jusqu'à disparition de l'adduit (I). La réaction est instantanée et la conversion de l'adduit (I), de formule TPOP₍₁₋ₓ₎TPPₓ, en adduit (II), de formule TPOP₍₁₋ₓ₎TPPₓ.Br₂ est quantitative.

Dans une troisième étape, l'alcool primaire est ajouté.

Cette troisième étape comprend tout d'abord une étape 3.1) de refroidissement, avant tout ajout d'alcool primaire, puis l'alcool primaire est ajouté (étape 3.2)).

Lors de l'étape 3.1), la température du milieu réactionnel est abaissée à une valeur comprise entre 0°C et -15°C, avantageusement comprise entre 0°C et -5°C.

Ensuite, lors de l'étape 3.2), un alcool primaire, tel que du cyclopropylméthanol ou du cyclobutylméthanol, est ajouté au milieu réactionnel.

Lors de cette étape 3.2), la température du mélange réactionnel est avantageusement maintenue à une valeur cible comprise entre 0°C et -15°C, avantageusement comprise entre 0°C et -5°C. Ainsi, l'alcool primaire est avantageusement ajouté lentement, c'est-à-dire à une vitesse telle que la température du milieu réactionnel se maintienne à la valeur cible.

Le milieu réactionnel est ensuite avantageusement maintenu sous agitation, avantageusement à une température correspondant à la valeur cible de l'étape 3.2), jusqu'à consommation complète de l'alcool primaire. Ensuite, le milieu réactionnel est avantageusement ramené à température ambiante.

Lors de l'étape 4), le composé bromé (A) est récupéré, par exemple en suivant les étapes décrites dans le brevet FR3016166.

Ainsi, par exemple, le milieu réactionnel, ramené à température ambiante, contenant le composé bromé (A) est soumis à une distillation selon des moyens connus de l'homme du métier. Par exemple, cette distillation peut être réalisée par chauffage sous pression réduite. La température de distillation peut être comprise entre 50 et 70°C, plus particulièrement aux environs de 65°C et ce à une pression comprise entre 1 mbar et 10 mbar, plus particulièrement aux environs de 5 mbar.

Après distillation, les fractions contenant le composé bromé (A) peuvent être lavées puis séchées.

Le lavage du composé bromé (A) peut être réalisé par tout moyen adéquat connu de l'homme du métier, par exemple à l'aide d'une solution tamponnée à pH8 telle qu'une solution de carbonate de sodium ou de potassium.

Le composé bromé (A) une fois lavé peut être séché à l'aide d'un dessicant et ce de manière connue de l'homme du métier. Le dessicant peut être choisi parmi les gels de silice, le chlorure de calcium, le chlorure de magnésium, les zéolites, le chlorure de lithium ou le bromure de lithium par exemple.

Le présent procédé présente l'avantage de fournir un composé bromé (A) avec un taux de pureté supérieur à 95%, particulièrement supérieur à 97%, plus particulièrement encore supérieur à 98%. Un tel taux de pureté est particulièrement avantageux pour ce qui concerne l'utilisation d'un composé bromé (A) lors d'étapes finales de synthèse de principes actifs pharmaceutiques pour lesquels une haute pureté est exigée. En particulier, pour le BMCP, le taux d'impuretés choisies parmi le 4-bromobut-1-ène et le bromocyclobutane est inférieur à 0,2%.

Les rendements obtenus grâce au procédé selon la présente invention, par rapport à l'alcool primaire de départ, sont supérieurs à 80%, ce qui est singulièrement avantageux dans un contexte industriel et particulièrement inattendu.

L'intérêt de ce procédé est d'arriver à des taux d'impuretés très faibles, pour le BMCP, inférieurs à 0,2% d'impuretés choisies parmi le 4-bromobut-1-ène et le bromocyclobutane, comme dans le brevet US 61913001 mais avec des rendements supérieurs à 80% par rapport à l'alcool primaire de départ. Par ailleurs, on bénéficiera du fait que l'adduit TPPₓTPOP₍₁₋ₓ₎ permet de faire réagir plus d'alcool primaire dans le même volume réactionnel qu'un procédé selon FR3016166 ou FR3010997. Par exemple pour x= 0,4 on obtient un gain de 8% de produit transformé dans le même réacteur et pour x=0,2 on obtient un gain de 4% de produit transformé dans le même réacteur. On obtient ainsi un procédé très productif et très sélectif, ce qui n'était absolument pas évident pour l'homme du métier.

Le procédé selon la présente invention ainsi que ses avantages pourront être mieux compris à l'aide des exemples illustratifs suivants.

### Exemples :

Les matières premières sont les matières premières trouvées chez Sigma Aldrich.

La méthode analytique consiste en une analyse par chromatographie en phase gazeuse (GC) sur un appareil HP 5890 Series II. La colonne chromatographique est une colonne Optima delta 6, 30 m, 0,25 mm, 0,25 µm.

Le four suit le profil de température suivant : Température initiale : 40°C, Temps initial 5 min. Gradient 5°/min ; Température finale : 125°C. Durée 15 min.

La température de l'injecteur est 250°C, celle du détecteur vaut 280°C, le volume injecté est de 1 µl et la pression de 6psi (ou 41368.5 Pa). La concentration de l'échantillon est de 75 g/l dans le tétrahydrofurane (THF).

Les réactions sont réalisées dans un réacteur de 20 I en verre à double enveloppe et les distillations sont effectuées au moyen d'une colonne verre de 10 plateaux théoriques.

### Exemple 1 : Préparation de l'adduit (I) TPPₓTPOP₍₁₋ₓ₎

Dans un réacteur propre, sec, sous azote, équipé d'un agitateur, sont chargés des quantités x de triphényl phosphine dans (1-x) parts en mol de triphényl phoshite à des températures Tx selon le tableau suivant :

**Tableau 1**

| | | | | | | | | |
|---|---|---|---|---|---|---|---|---|
| x | 0,1 | 0,2 | 0,3 | 0,4 | 0,5 | 0,6 | 0,7 | 0,8 |
| Tx (°C) | 25 | 25 | 25 | 25 | 45 | 50 | 55 | 55 |

Après homogénéisation, le mélange est ramené, le cas échéant, à température ambiante. Il peut être stocké à l'état liquide pour une utilisation ultérieure.

Le fait que le TPP soit solubilisé par le TPOP est caractéristiques d'interactions faibles entre les molécules de chaque espèce ce qui caractérise bien un adduit.

### Exemple 2 : Utilisation de l'adduit TPP_{0.2}TPOP_{(0.8)} pour la fabrication de BMCP

On introduit successivement dans le réacteur de 20L 4,63 kg de DMF (1,1 eqV), puis 4,53 kg de l'adduit (15,1mol). Sont ensuite introduits 2,74 kg (17 mol) de dibrome en maintenant la température en dessous de 12°C. La vitesse d'agitation est réglée en fonction de la fluidité du milieu réactionnel. En fin de coulée on obtient un milieu très épais avec un solide jaune en suspension.

La consigne de la double-enveloppe est ensuite réglée à -12°C, sont alors introduits 1,054 kg (14,6 mol) de cyclopropylméthanol de manière à ne pas dépasser une température de -5°C. A la fin de l'addition, on laisse revenir lentement à température ambiante. Puis, la consigne de la double-enveloppe est portée à 64°C pour la distillation, cette dernière est effectuée à une pression de 13 mbar en recueillant la première fraction de 24 à 30°C en tête de colonne, puis la seconde fraction de 30 à 40°C (reflux partiel). On recueille deux fractions F1 (1,77 kg) et F2 (340g). Les deux fractions, après lavage à l'eau carbonée puis séchage au moyen de CaCl₂, conduisent au BMCP (masse 1,664 kg, 12,05 mol) de pureté relative CPG de 98,9%, avec un rendement de 82%. Le taux de bromo butène mesuré est de 0.09%, le taux de bromocyclobutane est de 0.10%.

### Exemple comparatif 1 : procédé selon le brevet FR3016166:

Dans un réacteur propre, sec, sous azote, équipé d'un agitateur, sont chargés successivement 4,63 kg de DMF (5,1 eqV), puis 4,53 kg de triphénylphosphite. Sont ensuite introduits 2,34 kg de dibrome en maintenant la température en dessous de 12°C. La vitesse d'agitation est réglée en fonction de la fluidité du milieu réactionnel. En fin de coulée on obtient un milieu très épais avec un solide jaune en suspension. La consigne de la double-enveloppe est ensuite réglée à -12°C, sont alors introduits 0,96 kg de cyclopropylméthanol de manière à ne pas dépasser une température de -5°C. A la fin de l'addition, on laisse revenir lentement à température ambiante. Puis, la consigne de la double-enveloppe est portée à 64°C pour la distillation, cette dernière est effectuée à une pression de 13 mbar en recueillant la première fraction de 24 à 30°C en tête de colonne, puis la seconde fraction de 30 à 40°C (reflux partiel). On recueille deux fractions F1 (1,38 kg) et F2 (293g). Les deux fractions, après lavage à l'eau carbonée puis séchage au moyen de CaCl₂, conduisent à un produit final 2a (masse 1,316kg) de pureté relative CPG de 98,7% avec un rendement de 73%.

La comparaison entre l'exemple 2 et l'exemple comparatif 1 met en évidence : un rendement supérieur de 9%, un taux de matière première engagé supérieur de 4% ce qui contribue à faire passer la production par litre de réacteur utile de 65,8 g/l à 83,2g/l.

### Exemple 3 : Caractérisation de la réactivité spécifique des adduits (I) avec le dibrome

De manière à mettre en évidence la différence de comportement de la TPP ou du TPOP purs par rapport aux adduits (I) de l'invention nous avons conduit l'expérience suivante.

Essais A : Dans un tube RMN nous avons ajouté 0,5 ml de DMF deutéré anhydre puis 40 mg de TPP_{0.2}TPOP_{0.8}. Le tube a été refroidi à 0°C et nous avons ensuite rajouté 11mg de dibrome. Nous avons réalisé ensuite une RMN du phosphore et le déplacement chimique observé est de 3ppm avec un signal assez large caractéristiques d'équilibres chimiques.

Essais B : Dans un tube RMN nous avons ajouté 0,5 ml de DMF deutéré anhydre puis 40 mg de TPP. Le tube a été refroidi à 0°C et nous avons ensuite rajouté 11mg de dibrome. Nous avons réalisé ensuite une RMN du phosphore. Le déplacement chimique observé est de -2 ppm.

Essais C : Dans un tube RMN nous avons ajouté 0,5 ml de DMF deutéré anhydre puis 40 mg de TPOP. Le tube a été refroidi à 0°C et nous avons ensuite rajouté 11mg de dibrome. Nous avons réalisé ensuite une RMN du phosphore. Le déplacement chimique observé est de 7ppm.

Ces trois expériences montrent que l'adduit TPP_{0.2}TPOP_{0.8}conduit par réaction avec le dibrome à un adduit différent de ceux obtenus par le TPOP pur ou le TPP pur. Cette expérience montre que les composés mixtes TPPBr₂-TPOPBr₂ sont en interaction et forment des espèces mixtes en solution ce qui peut expliquer la réactivité différente des adduits (I) par rapport aux espèces pures TPP ou TPOP.

### Exemple 4 :

En reprenant les conditions expérimentales de l'exemple 1, les rendements et impuretés sont évalués selon différentes valeurs de X.

Pour X supérieur à 0,4, le mélange de triphénylphosphine dans la triphénylphosphite présente, à température ambiante, des difficultés de solubilité. Souhaitant rester à température ambiante, la valeur de X=0,4 a représenté la limite des présents essais. X = 0 représente le procédé selon l'art antérieur. Des essais au laboratoire conduisent aux résultats suivants :

| | Alcool de départ | Produit Fini | 4-bromobut-1-ène | Bromocyclobuta ne |
|---|---|---|---|---|
| X = 0 | 0.51% | 86% | ND | 0.25% |
| X = 0.1 | 2.16% | 88% | 0.09% | 0.24% |
| X = 0.2 | 2.09% | 84% | ND | ND |
| X = 0.3 | 3.59% | 88% | ND | ND |

| | | | | |
|---|---|---|---|---|
| ND : non détecté | | | | |

La teneur en bromo cyclopropane produit étant comparable mais la conversion un peu plus basse avec l'ajout de triphénylphosphine, cela implique qu'à conversion égale, la pureté est supérieure. De plus, les impuretés 4-bromobut-1-ène et le bromocyclobutane sont absentes à plus haute teneur en triphénylphosphine.

## Revendications

1. Adduit de formule générale (I)
TPPₓTPOP₍₁₋ₓ₎ (I)
où
- TPP désigne la triphényl phosphine ;
- TPOP désigne le triphényl phosphite ; et
- x est un nombre réel compris entre 0,05 et 0,9.

2. Adduit selon la revendication 1, pour lequel x est compris entre 0,05 et 0,5.

3. Procédé de préparation de l'adduit selon la revendication 1 ou 2, dans lequel on mélange x part en moles de triphényl phosphine et (1-x) part en moles de triphényl phosphite à une température Tx comprise entre 20°C et 50°C.

4. Procédé de préparation selon la revendication 3, dans lequel Tx vaut 25°C et x<0,5.

5. Utilisation de l'adduit selon la revendication 1 ou 2 ou de l'adduit obtenu par le procédé selon la revendication 3 ou 4 en tant qu'agent liquide de bromation d'alcool primaire.

6. Utilisation selon la revendication précédente, dans laquelle l'alcool primaire comprend un cycle hydrocarboné à 3 ou 4 atomes de carbone, en particulier l'alcool primaire est le cyclopropyl-méthanol ou le cyclobutyl-méthanol.

7. Procédé de préparation d'un composé bromé par bromation d'alcool primaire comprenant les étapes suivantes :
1. mélanger (a) 1 équivalent molaire de l'adduit (I) selon l'invention ou de l'adduit obtenu par le procédé selon l'invention et (b) 1 à 3 volumes de solvant polaire ; puis
2. ajouter le dibrome :
2.1 refroidir sous agitation la solution obtenue suite à l'étape 1) à une température comprise entre 0°C et 12°C, puis
2.2 à la solution refroidie obtenue suite à l'étape 2.1), ajouter entre 1,05 et 1,25 équivalent molaire de dibrome en maintenant la température du milieu réactionnel en dessous de 12°C ; puis
3. ajouter l'alcool primaire :
3.1 suite à l'étape 2.2), abaisser la température du milieu réactionnel à une valeur comprise entre 0°C et -15°C ;
3.2 au milieu réactionnel refroidi obtenu suite à l'étape 3.1), ajouter entre 0,95 et 1 équivalent molaire d'alcool primaire, puis
4. récupérer le composé bromé.

8. Procédé selon la revendication 7, dans lequel le solvant polaire est choisi parmi le diméthyl formamide, le diméthyl sulfoxyde, le sulfolane, le dichlorométhane, le tétrahydrofurane, le diméthylglycol éther, le dichloroéthane, l'acétonitrile, et leurs mélanges.

9. Procédé selon la revendication 7 ou 8, dans lequel lors de l'étape 2.2) le milieu réactionnel est maintenu à une température comprise entre 0°C et 12°C.

10. Procédé selon l'une quelconque des revendications 7 à 9, dans lequel lors de l'étape 3.2) le milieu réactionnel est maintenu à une température comprise entre 0°C et -15°C, avantageusement comprise entre 0°C et -5°C.

11. Procédé selon l'une quelconque des revendications 7 à 10, dans lequel l'alcool primaire comprend un cycle hydrocarboné à 3 ou 4 atomes de carbone.

12. Procédé selon l'une quelconque des revendications 7 à 11, dans lequel l'alcool primaire est le cyclopropyl carbinol ou le cyclobutyl carbinol.

13. Procédé selon la revendication 12, dans lequel l'alcool primaire est le cyclopropyl carbinol et le composé bromé récupéré comprend, en poids par rapport au poids total, moins de 0,2% d'impuretés choisies parmi le 4-bromobut-1-ène et le bromocyclobutane.

14. Adduit de formule générale (II) :
TPPₓTPOP₍₁₋ₓ₎Br₂₍ₚ₎ (II)
où
- TPP désigne la triphényl phosphine ;
- TPOP désigne le triphényl phosphite ;
- x est un nombre réel compris entre 0,05 et 0,9 ; et
- p est égal à 1 ou 2.

15. Utilisation de l'adduit selon la revendication 14 pour la bromation d'alcool primaire, en particulier le cyclopropylméthanol ou le cyclobutylméthanol.

## Patentansprüche

1. Addukt der allgemeinen Formel (I),
TPPₓTPOP₍₁₋ₓ₎ (I),
in der
- TPP für Triphenylphosphin steht;
- TPOP für Triphenylphosphit steht und
- x eine reelle Zahl ist, die zwischen 0,05 und 0,9 liegt.

2. Addukt nach Anspruch 1, wobei x zwischen 0,05 und 0,5 liegt.

3. Verfahren zur Herstellung des Addukts nach Anspruch 1 oder 2, wobei x Teile in Mol Triphenylphosphin und (1-x) Teile in Mol Triphenylphosphit bei einer Temperatur Tx, die zwischen 20 °C und 50 °C liegt, gemischt werden.

4. Herstellungsverfahren nach Anspruch 3, wobei Tx gleich 25 °C ist und x < 0,5.

5. Verwendung des Addukts nach Anspruch 1 oder 2 oder des durch das Verfahren nach Anspruch 3 oder 4 erhaltenen Addukts als flüssiges Mittel zur Bromierung eines primären Alkohols.

6. Verwendung nach dem vorhergehenden Anspruch, wobei der primäre Alkohol einen Kohlenwasserstoffring mit 3 oder 4 Kohlenstoffatomen umfasst, insbesondere wobei der primäre Alkohol Cyclopropylmethanol oder Cyclobutylmethanol ist.

7. Verfahren zur Herstellung einer bromierten Verbindung durch Bromierung eines primären Alkohols, umfassend die folgenden Schritte:
1. Mischen von (a) 1 Moläquivalent des erfindungsgemäßen Addukts (I) oder des durch das erfindungsgemäße Verfahren erhaltenen Addukts und (b) 1 bis 3 Volumina eines polaren Lösungsmittels und
2. Zugeben von Dibrom:
2.1 Kühlen der nach Schritt 1) erhaltenen Lösung unter Rühren bei einer Temperatur, die zwischen 0 °C und 12 °C liegt, dann
2.2 Zugeben von zwischen 1,05 und 1,25 Moläquivalenten Dibrom zu der nach Schritt 2.1) erhaltenen gekühlten Lösung, wobei die Temperatur des Reaktionsmediums unter 12 °C gehalten wird; dann
3. Zugeben des primären Alkohols:
3.1 nach Schritt 2.2) Senken der Temperatur des Reaktionsmediums auf einen Wert, der zwischen 0 °C und -15 °C liegt;
3.2 Zugeben von zwischen 0,95 und 1 Moläquivalent eines primären Alkohols in das nach Schritt 3.1) erhaltene gekühlte Reaktionsmedium, dann
4. Gewinnen der bromierten Verbindung.

8. Verfahren nach Anspruch 7, wobei das polare Lösungsmittel aus Dimethylformamid, Dimethylsulfoxid, Sulfolan, Dichlormethan, Tetrahydrofuran, Dimethylglykolether, Dichlorethan, Acetonitril und deren Gemischen ausgewählt ist.

9. Verfahren nach Anspruch 7 oder 8, wobei während des Schritts 2.2) das Reaktionsmedium auf einer Temperatur gehalten wird, die zwischen 0 °C und 12 °C liegt.

10. Verfahren nach einem der Ansprüche 7 bis 9, wobei während des Schritts 3.2) das Reaktionsmedium auf einer Temperatur gehalten wird, die zwischen 0 °C und -15 °C liegt, vorteilhaft zwischen 0 °C und -5°C liegt.

11. Verfahren nach einem der Ansprüche 7 bis 10, wobei der primäre Alkohol einen Kohlenwasserstoffring mit 3 oder 4 Kohlenstoffatomen umfasst.

12. Verfahren nach einem der Ansprüche 7 bis 11, wobei der primäre Alkohol Cyclopropylcarbinol oder Cyclobutylcarbinol ist.

13. Verfahren nach Anspruch 12, wobei der primäre Alkohol Cyclopropylcarbinol ist und die gewonnene bromierte Verbindung, in Gewicht bezogen auf das Gesamtgewicht, weniger als 0,2 % Verunreinigungen umfasst, die aus 4-Brombut-1-en und Bromcyclobutan ausgewählt sind.

14. Addukt der allgemeinen Formel (II):
TPPₓTPOP₍₁₋ₓ₎Br₂₍ₚ₎ (II),
in der
- TPP für Triphenylphosphin steht;
- TPOP für Triphenylphosphit steht und
- x eine reelle Zahl ist, die zwischen 0,05 und 0,9 liegt; und
- p gleich 1 oder 2 ist.

15. Verwendung des Addukts nach Anspruch 14 zur Bromierung eines primären Alkohols, insbesondere Cyclopropylmethanol oder Cyclobutylmethanol.

## Claims

1. Adduct of general formula (I)
TPPₓTPOP₍₁₋ₓ₎ (I)
wherein
- TPP designates triphenylphosphine;
- TPOP designates triphenyl phosphite; and
- x is a real number comprised between 0.05 and 0.9.

2. Adduct according to claim 1, for which x is comprised between 0.05 and 0.5.

3. Preparation method for the adduct according to claim 1 or 2, wherein x parts by mole of triphenylphosphine and (1-x) parts by mole of triphenyl phosphite are mixed at a temperature Tx comprised between 20°C and 50°C.

4. Preparation method according to claim 3, wherein Tx is 25°C and x<0.5.

5. Use of the adduct according to claim 1 or 2 or the adduct obtained by the method according to claim 3 or 4 as a liquid bromination agent for primary alcohol.

6. Use according to the preceding claim, wherein the primary alcohol comprises a hydrocarbon ring with 3 or 4 carbon atoms, in particular the primary alcohol is cyclopropylmethanol or cyclobutylmethanol.

7. Preparation method for a brominated compound by bromination of primary alcohol comprising the following steps:
1. Mixing (a) 1 molar equivalent of adduct (I) according to the invention or the adduct obtained by the method according to the invention and (b) 1 to 3 volumes of polar solvent; then
2. Adding the dibromine:
2.1 cool with stirring the solution obtained in step 1) to a temperature comprised between 0°C and 12°C, then
2.2 to the cooled solution obtained after step 2.1), add between 1.05 and 1.25 molar equivalents of dibromine while keeping the reaction medium temperature below 12°C; then
3. Adding the primary alcohol:
3.1 following step 2.2), lower the reaction medium temperature to a value comprised between 0°C and -15°C;
3.2 to the cooled reaction medium obtained following step 3.1), add between 0.95 and 1 molar equivalent of primary alcohol, then
4. Recovering brominated compound.

8. Method according to claim 7, wherein the polar solvent is chosen from among dimethylformamide, dimethyl sulfoxide, sulfolane, dichloromethane, tetrahydrofuran, dimethyl glycol ether, dichloroethane, acetonitrile, and mixtures thereof.

9. Method according to claim 7 or 8, wherein during step 2.2) the reaction medium is maintained at a temperature comprised between 0°C and 12°C.

10. Method according to any of claims 7 to 9, wherein during step 3.2) the reaction medium is maintained at a temperature comprised between 0°C and -15°C, advantageously comprised between 0°C and -5°C.

11. Method according to any of claims 7 to 10 wherein the primary alcohol comprises a hydrocarbon ring with 3 or 4 carbon atoms.

12. Method according to any of claims 7 to 11 wherein the primary alcohol is cyclopropyl carbinol or the cyclobutyl carbinol.

13. Method according to claim 12, wherein the primary alcohol is cyclopropyl carbinol and the brominated compound recovered comprises, by weight relative to the total weight, less than 0.2% of impurities chosen among 4-bromobut-1-ene and bromocyclobutane.

14. Adduct of general formula (II)
TPPₓTPOP₍₁₋ₓ₎Br₂₍ₚ₎ (II)
wherein
- TPP designates triphenylphosphine;
- TPOP designates triphenyl phosphite;
- x is a real number comprised between 0.05 and 0.9; and
- p is equal to 1 or 2.

15. Use of the adduct according to claim 14 for bromination of primary alcohol, in particular cyclopropylmethanol or cyclobutylmethanol.
